# EUROPÄISCHE PATENTSCHRIFT

(11) **EP 0 975 578 B1**
(45) Veröffentlichungstag und Bekanntmachung des Hinweises auf die Patenterteilung: **03.04.2002**
(21) Anmeldenummer: 98917111.1
(22) Anmeldetag: 06.04.1998
(51) Int. Cl.: C07C 209/36, B01J 23/656

(54) **HERSTELLUNG VON AROMATISCHEN AMINEN MITTELS NEUER HYDRIERKATALYSATOREN**
PRODUCTION OF AROMATIC AMINES BY MEANS OF NOVEL HYDROGENATION CATALYSTS
PRODUCTION D'AMINES AROMATIQUES AU MOYEN DE NOUVEAUX CATALYSEURS D'HYDROGENATION

(30) Priorität: 16.04.1997 DE 19715746
(43) Veröffentlichungstag der Anmeldung: 02.02.2000
(73) Patentinhaber: BAYER AG, 51368 Leverkusen (DE)
(72) Erfinder: LANGER, Reinhard, D-47800 Krefeld (DE); BUYSCH, Hans-Josef, D-47809 Krefeld (DE); GALLUS, Manfred, D-47800 Krefeld (DE); JENTSCH, Joerg, Dietrich, D-45468 Mülheim an der Ruhr (DE)
(86) Internationale Anmeldenummer: EP9801985
(87) Internationale Veröffentlichungsnummer: WO9846557

(56) Entgegenhaltungen:
- EP-A- 0 011 090
- EP-A- 0 748 790
- DE-A- 2 214 056
- DE-A- 19 620 542
- US-A- 3 960 964

## Beschreibung

Die vorliegende Erfindung betrifft die Herstellung von aromatischen Aminen durch katalytische Hydrierung von entsprechenden aromatischen Nitroverbindungen in der Gasphase mittels neuer palladiumhaltigen Trägerkatalysatoren.

Die Hydrierung von Nitrobenzol zu den entsprechenden aromatischen Aminen in der Gasphase an ortsfest angebrachten Palladium-Katalysatoren auf keramischen Trägern ist bekannt. So wird in DE-A 2 849 002 ein Verfahren zur Reduktion von Nitroverbindungen in Gegenwart von palladiumhaltigen Mehrkomponenten-Trägerkatalysatoren in gekühlten Rohrreaktoren beschrieben. Der Kontakt besteht im wesentlichen aus 1 bis 20 g Palladium, 1 bis 20 g Vanadium und 1 bis 20 g Blei pro Liter α-Al₂O₃. Nachteilig bei den in der erwähnten Patentveröffentlichung beschriebenen Gasphasen-hydrierungen ist die geringe Belastung (spezifische Belastung) der Katalysatoren. Die angegebenen Belastungen betragen ca. 0,4 bis 0,5 kg/l x h. Die Belastung ist dabei definiert als die Menge an Nitroaromaten in kg, die pro Liter Katalysatorschüttung innerhalb einer Stunde umgesetzt wird. Verbunden mit der geringen Katalysatorbelastung ist eine nicht befriedigende Raumzeitausbeute bei großtechnischen Verfahren zur Herstellung von aromatischen Aminen. Ferner sind die Selektivitäten zu Beginn einer Fahrperiode deutlich niedriger als gegen Ende, was zu Ausbeuteverlusten und Schwierigkeiten bei der Aufarbeitung des Rohproduktes führt.

Aufgabe der vorliegenden Erfindung war es nun ein Verfahren zur Herstellung von aromatischen Aminen durch katalytische Hydrierung entsprechender aromatischer Nitroverbindungen zur Verfügung zu stellen, das ohne Probleme im großtechnischen Maßstab durchzuführen ist, das eine hohe Raumzeitausbeute ermöglicht und das eine Selektivität bezüglich der gewünschten Amine von ≥90 % auch zu Beginn einer Fahrperiode aufweist.

Gegenstand der vorliegenden Erfindung ist daher ein Verfahren zur Herstellung von aromatischen Aminen durch katalytische Hydrierung von entsprechenden aromatischen Nitroverbindungen in der Gasphase, das dadurch gekennzeichnet ist, das man die Hydrierung in Gegenwart von Katalysatoren, die neben Palladium, Vanadin und Blei noch zusätzlich Rhenium auf einem keramischen Trägermaterial mit einer BET-Oberfläche von weniger als 40 m³/g enthalten, bei Temperaturen von 180 bis 500°C im Katalysatorbett und einem molaren Verhältnis von Wasserstoff zur Nitrogruppe oder Nitrogruppen von 3:1 bis 30:1 durchführt.

Als aromatische Nitroverbindungen, die nach dem erfindungsgemäßen Verfahren hydriert werden können, kommen insbesondere solche der nachstehenden Formel in Frage: 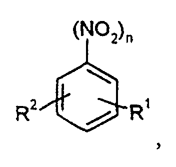 in der
- R¹ und R²: gleich oder verschieden sind und für Wasserstoff oder C₁-C₄-Alkyl, insbesondere Methyl und Ethyl, stehen und
- n: 1 oder 2 bedeutet.

Bevorzugt werden Nitrobenzol oder die isomeren Nitrotoluole nach dem erfindungsgemäßen Verfahren hydriert.

Als keramische Trägermaterialien für die erfindungsgemäßen Katalysatoren eignen sich im Prinzip alle keramischen Feststoffe mit BET-Oberflächen von weniger als 40 m²/g, bevorzugt weniger als 20 m²/g, insbesondere weniger als 10 m²/g. Insbesondere sind als keramische Feststoffe geeignet: Metalloxide und/oder Metallmischoxide der Elemente Magnesium, Aluminium, Silicium, Germanium, Zirkonium und Titan, bevorzugt wird als Trägermaterial α-Aluminiumoxid eingesetzt.

Überraschenderweise wurde gefunden, daß Kontakte, welche neben Palladium, Vanadium und Blei noch Re auf die obengenannten Trägermaterialien, insbesondere α-Aluminiumoxid, schalenförmig niedergeschlagen enthalten, besonders standfeste, hoch belastbare und selektive Katalysatoren sind. Besonders hervorzuheben sind daher Katalysatoren, die a) 1 bis 50 g Palladium, b) 1 bis 50 g, Vanadium, c) 1 bis 20 g Blei und d) 1 bis 20 g Rhenium pro Liter oxidischen Trägermaterials enthalten, wobei das Trägermaterial eine Oberfläche von weniger als 40, bevorzugt weniger als 20, insbesondere weniger als 10 m²/g aufweist. Besonders bevorzugt werden Katalysatoren eingesetzt, die 10 bis 50 g Pd, 10 bis 50 g V, 8 bis 16 g Pb und 8 bis 16 g Re auf α-Al₂O₃ enthalten.

Die Herstellung von Edelmetall-Träger-Katalysatoren ist prinzipiell bekannt. Dabei hat es sich als Vorteil erwiesen, wenn die aktiven Komponenten des Katalysators möglichst nahe an der Trägerformkörperoberfläche als scharfe Zone niedergeschlagen vorliegen und das Innere des Trägermaterials kein Metall enthält. Die Katalysatoren können dabei mit oder ohne Vorbehandlung des Trägermaterials mit einer Base hergestellt werden.
Bevorzugt wird das Trägermaterial vor den Tränkungen mit einer Alkali- oder Erdalkalihydroxid-Lösung getränkt.

Die Metalle können einzeln oder als Mischung in Form von Lösungen ihrer Salze auf den Träger aufgebracht werden. Als Salze sind beispielsweise die Halogenide, Acetate, Carbonate, Hydrogencarbonate, Sulfate, Phosphate, Oxalate, Formiate, Oxide und Hydroxide geeignet. Nach jedem Tränkgang und/oder zum Abschluß wird eine Reduktion durchgeführt, zu der Wasserstoff, Hydrazin und/oder Ameisensäure eingesetzt wird.

Prinzipiell können die erfindungsgemäßen Trägerkatalysatoren jede beliebige Form aufweisen, wie Kugeln, Stäbchen, Raschigringe, Granulat oder Tabletten. Bevorzugt werden Formkörper benutzt, deren Schüttungen einen niedrigen Strömungswiderstand bei gutem Gas-Oberflächen-Kontakt aufweisen, wie Raschigringe, Sattelkörper, Wagenräder und/oder Spiralen. Die erfindungsgemäßen Katalysatoren können rein oder in Verdünnung mit anderen inerten Füllkörpern z.B. aus Glas, Keramik oder Metall eingesetzt werden. Die Katalysatorschüttung kann bis zu 90 Gew.-%, bevorzugt bis zu 75 Gew.-%, insbesondere bis zu 50 Gew.-%, aus inerten Füll- oder Trägermaterial bestehen. Die Schüttung kann dabei einen Verdünnungsgradienten der Art aufweisen, daß die Verdünnung in Strömungsrichtung abnimmt. Die Schüttung kann an der Aufströmoberfläche zwischen 50 bis 90 Gew.-% Füllmaterial enthalten und am Ausströmende zwischen 80 und 100 Gew.-% aus reinem Trägerkatalysator bestehen.

Bei dem erfindungsgemäßen Verfahren werden die Trägerkatalysatoren in Mengen von 40 bis 80 %, bezogen auf das Gesamtvolumen Schüttung, bevorzugt in Mengen von 50 bis 70 %, eingesetzt.

Das molare Verhältnis von Wasserstoff zu eingesetzten Nitroverbindungen (Nitrogruppen) bei dem erfindungsemäßen Verfahren beträgt 4:1 bis 20:1, insbesondere 5:1 bis 10:1. Hierbei kann die Wasserstoffkonzentration durch Zumischen von Inertgasen, wie Stickstoff, Helium, Argon und/oder Wasserdampf, herabgesetzt werden. Bevorzugt wird Stickstoff als Inertgas zugemischt. Pro Mol Wasserstoff können bis zu 10 mol, bevorzugt bis zu 3 mol, insbesondere bis zu 1 mol, inertes Trägergas zugemischt werden.

Die Verdünnung mit einem inerten Trägergas wird bevorzugt zu Beginn einer Fahrperiode mit frischem Katalysator und nach dem Regenerieren des Katalysators durch Abbrennen mit Luft und Reduzierung mit Wasserstoff angewendet. Bevorzugt wird in den ersten 300 Stunden, besonders bevorzugt in den ersten 200 Stunden, insbesondere in den ersten 100 Stunden, nach dem Wiederanfahren mit Inertgas verdünnt.

Die Regenerierung desaktivierter Katalysatorschüttungen erfolgt mit Stickstoff/Sauerstoff-Mischungen bei Temperaturen von 200 bis 400°C, bevorzugt bei 250 bis 350°C, am Katalysator selbst. Dabei wird im allgemeinen bei N₂-Gehalten von 90 bis 99 % im Gasstrom begonnen und der O₂-Gehalt während des Abbrennes schrittweise auf den Gehalt reiner Luft angehoben, gegebenenfalls können am Ende der Regenerierung mit reinem Sauerstoff hartnäckige Verkokungen abgebrannt werden. Anstelle von Stickstoff können auch andere inerte Trägergase zu Sauerstoff oder Luft zugemischt werden, wie Argon, Helium und/oder Wasserdampf. Das erfindungsgemäße Verfahren wird bevorzugt bei Temperaturen von 200 bis 460°C, insbesondere bei 220 bis 440°C, betrieben. Dabei kann es von Vorteil sein, wenn die Temperatur des Kühlmediums im erfindungsgemäßen Verfahren während eines Betriebszyklus kontinuierlich oder schrittweise angehoben wird.

Der Druck bei dem erfindungsgemäßen Verfahren beträgt 0,5 bis 5, bevorzugt 1 bis 3 bar.

Die Belastungen der erfindungsgemäßen Katalysatoren an eingesetzter aromatischer Nitroverbindung sollte kontinuierlich oder stufenweise von 0,01 bis 0,2 auf 0,5 bis 5,0 kg/l erhöht werden, wobei die maximale Belastung innerhalb von 10 bis 1 000 Stunden erreicht wird.

Für die erfindungsgemäßen Katalysatoren ist besonders günstig, wenn die Belastung an eingesetzten aromatischen Nitroverbindungen innerhalb eines gewissen Zeitraums kontinuierlich oder stufenweise auf den gewünschten Belastungswert erhöht wird. Bevorzugt ist daher eine Arbeitsweise in der die Belastung des Katalysators innerhalb der ersten 500, insbesondere der ersten 300, ganz besonders bevorzugt der ersten 200 Stunden kontinuierlich oder stufenweise von 0,01 bis 0,5, bevorzugt 0,1 bis 0,4, insbesondere 0,15 bis 0,3 auf 0,6 bis 5,0, insbesondere von 0,6 bis 3,0, ganz besonders bevorzugt 0,6 bis 2,0 kg/l x h erhöht wird.

Die hohe Endbelastung wird bis zum Durchbruch von unumgesetztem Edukt konstant gehalten. Wenn die Eduktkonzentration am Reaktorende einen zu hohen Wert annimmt kann die Temperatur des Wärmeträgers angehoben werden und/oder die Belastung an Edukt gesenkt werden. um eine Produktionsunterbrechung zur Regeneration des Katalysators herauszuzögern.

Die technische Verwirklichung eines Verfahrens mit den erfindungsgemäßen Katalysatoren kann beispielsweise wie folgt erfolgen: Ein Kreisgastrom im wesentlichen bestehend aus Wasserstoff und wenig Wasser wird verdichtet, um die Strömungswiderstände der Anlage zu überwinden. Mittels Gegenstromwärmetauschung wird der Gasstrom aufgeheizt, wobei die Wärme z.B. dem Kreisgasstrom vor der Kondensation der Produkte entnommen wird. Der Kreisgastrom wird auf die gewünschte Temperatur gebracht. Im Frischwasserstoff, der den verbrauchten ersetzt, wird der zu hydrierende Nitroaromat verdampft, überhitzt und anschließend werden die beiden Gasströme vermischt. Das Gasgemisch wird in einem thermostatisierten Reaktor mit stationär angeordneten Katalysator eingeleitet. Die freiwerdende Reaktionswärme wird mittels eines Wärmeträgers dem Reaktor entnommen. Der den Reaktor verlassende Produktstrom wird zum Aufheizen des Kreisgasstromes genutzt und dann bis zur Kondensation von gebildeteten Anilin und Wasser abgekühlt. Die Flüssigkeiten werden ausgeschleust, ebenso eine kleine Menge Kreisgas zum Entfernen von Gasen, z.B. Stickstoff, die sich sonst anreichern würden. Das Kreisgas wird anschließend wieder dem Verdichter zugeführt.

In einer bevorzugten Ausgestaltung wird der erfindungsgemäße Katalysator als Schüttung in einen Reaktor vom Lindetyp (Wärmeträger fließt innerhalb der Reaktorrohre und der Katalysator ist außerhalb der Wärmeträgerrohre angeordnet) eingebracht und wie oben beschrieben verfahren. Dabei wird frischer oder frisch regenerierter Katalysator in den ersten Stunden mit Stickstoff-Wasserstoff-Mischungen betrieben. Die Vorteile dieser bevorzugten Betriebsweise sind: hohe Selektivitäten und lange Zeiträume zwischen den Regenerierungen auch nach vielen Produktionszyklen.

Als Reaktoren können für das erfindungsgemäße Verfahren alle bekannten Reaktoren eingesetzt werden, die für Gasphasenreaktionen mit gekühlten stationären Katalysatorschüttungen geeignet sind. Geeignet sind z.B. Rohrbündelreaktoren, in denen sich der Katalysator innerhalb von Wärmeträger umspülten Rohren befindet und Reaktoren, in denen umgekehrt der Wärmeträger innerhalb der Rohre fließt und der Katalysator sich außerhalb der Rohre befindet. Beispielsweise sind solche Reaktoren aus DE-A 2 848 014 und 3 007 202 bekannt.

Als besonders vorteilhaft haben sich Reaktoren für das erfindungsgemäße Verfahren erwiesen, in denen der Wärmeträger innerhalb der Rohre fließt und der Katalysator sich außerhalb der Wärmeträgerrohre befindet (Reaktoren vom Lindetyp). In diesen Reaktoren werden, relativ zu konventionellen Rohrbündelreaktoren, über viele Regenerationszyklen konstante Laufzeiten zwischen den Regenerierungen beobachtet.

Die Länge der Katalysatorschüttung in Strömungsrichtung beim erfindungsgemäßen Verfahren liegt bei 0,5 bis 20, bevorzugt 1 bis 10, besonders bevorzugt bei 2 bis 6 m.

Die Schüttungslänge kann ggf. auch durch mehrere hintereinander geschaltete Reaktoren erreicht werden.

Ein weiterer Gegenstand der vorliegenden Erfindung sind Hydrierkatalysatoren, die neben Palladium, Vanadin und Blei noch zusätzlich Rhenium auf einem keramischen Träger mit einer BET-Oberfläche von weniger als 40 m³/g enthalten und wobei der Gehalt an Palladium 1 bis 50 g, der Gehalt an Vanadium 1 bis 50 g, der Gehalt an Blei 1 bis 20 g und der Gehalt an Rhenium insgesamt 1 bis 20 g pro Liter keramischen Trägermaterials beträgt.

Als Trägermaterialien kommen insbesondere die zuvor beschriebenen Trägermaterialien in Frage, insbesondere α-Aluminiumoxid.

Das erfindungsgemäße Verfahren mit den neuen Katalysatoren zeichnet sich insbesondere durch hohe Raumzeitausbeuten aus, verbunden mit einer Verringerung der benötigten Apparategrößen und einer deutlich erhöhten Produktivität der Katalysatoren. Damit kann eine erhebliche Produktionssteigerung in bestehenden Anlagen erzielt werden. Darüber hinaus zeichnet sich das erfindungsgemäße Verfahren durch eine besonders hohe Selektivität bezüglich des Hydrierproduktes aus.

### Beispiele

### Katalysatorpräparation

### Beispiel 1 (Vergleichsbeispiel)

Ein Liter eines α-Al₂O₃-Trägers in Kugelform mit 3 bis 5 Millimetern Durchmesser, einer BET-Oberfläche von 9,8 m²/g, einer Saugfähigkeit von 45,1 ml Wasser pro 100 g Träger und einem Schüttgewicht von 812 g/l wurde mit 366 ml einer 10,8 g (entsprechend 0,27 Grammäquivalenten) NaOH enthaltenden wäßrigen Lösung imprägniert. Die Lösung wurde innerhalb weniger Minuten völlig vom Träger aufgenommen.

Der feuchte Träger wurde in einem warmen aufsteigenden starken Luftstrom getrocknet. Die Trockenzeit bis zur Gewichtskonstanz betrug ungefähr 15 Minuten. Der Restfeuchtegehalt lag nach dem Abkühlen etwa bei 1 % der Saugfähigkeit des Trägers.

Der so vorbehandelte trockene Träger wurde seiner Saugfähigkeit entsprechend mit 366 ml einer wäßrigen Natriumtetrachloropalladat-Lösung, die 9 g Palladium (entsprechend 0,169 Grammäquivalenten) enthielt, getränkt und 15 Minuten stehen gelassen. Zur Reduktion der auf dem Träger abgeschiedenen Palladiumverbindung zu metallischem Palladium wurde der Katalysator mit 400 ml einer 10 %igen wäßrigen Hydrazinhydrat-Lösung überschichtet und 2 Stunden stehen gelassen. Danach wurde der Katalysator mit vollentsalztem Wasser gründlich gespült, bis keine Ionen der bei der Katalysatorherstellung verwendeten Verbindungen im Waschwasser mehr nachweisbar waren, was nach ca. 10 Stunden der Fall war.

Anschließend wurde wieder in einem starken warmen aufsteigenden Luftstrom bis zur Gewichskonstanz getrocknet.

Der Pd-haltige Katalysator wurde anschließend mit 366 ml einer wäßrigen, 9 g Vanadium als Vanadyloxalat enthaltenden Lösung getränkt. Die Trockung des Trägers im Warmluftstrom erfolgte wie oben angegeben. Anschließend wurde der Katalysator in einem Rohrofen bei 300°C 6 Stunden thermisch behandelt, wobei das Oxalat zersetzt wurde.

Abschließend wurde der Katalysator mit 366 ml einer wäßrigen, 3 g Blei als Bleiacetat enthaltenden Lösung getränkt und wiederum im aufsteigenden Luftstrom getrocknet.

Der fertige Katalysator enthielt 9 g Pd, 9 g Vanadium und 3 g Blei und entsprach dem Katalysator aus Beispiel 1 in DE-OS 2 849 002.

### Beispiel 2

Ein Liter eines α-Al₂O₃-Trägers in Kugelform mit 3 bis 5 Millimetern Durchmesser, einer BET-Oberfläche von 9,8 m²/g, einer Saugfähigkeit von 45,1 ml Wasser pro 100 g Träger und einem Schüttgewicht von 831 g/l wurde mit 367 ml einer 48 g (entsprechend 1,2 Grammäquivalenten) NaOH enthaltenden wäßrigen Lösung imprägniert. Die Lösung wurde innerhalb weniger Minuten völlig vom Träger aufgenommen.

Der feuchte Träger wurde in einem warmen aufsteigenden starken Luftstrom getrocknet.
Die Trockenzeit bis zur Gewichtskonstanz betrug ungefähr 15 Minuten. Der Restfeuchtegehalt lag nach dem Abkühlen etwa bei 1 % der Saugfähigkeit des Trägers.

Der so vorbehandelte trockene Träger wurde seiner Saugfähigkeit entsprechend mit 352 ml einer wäßrigen Natriumtetrachloropalladat-Lösung, die 40 g Palladium (entsprechend 0,751 Grammäquivalenten) enthielt, getränkt und 24 Stunden stehen gelassen. Zur Reduktion der auf dem Träger abgeschiedenen Palladiumverbindung zu metallischem Palladium wurde der Katalysator mit 400 ml einer 10 %igen wässigen Hydrazinhydrat-Lösung überschichtet und 2 Stunden stehen gelassen. Danach wurde der Katalysator mit vollentsalztem Wasser gründlich gespült, bis keine Ionen der bei der Katalysatorherstellung verwendeten Verbindungen im Waschwasser mehr nachweisbar waren, was nach ca. 10 Stunden der Fall war.

Anschließend wurde wieder in einem starken warmen aufsteigenden Luftstrom bis zur Gewichskonstanz getrocknet.

Der Pd-haltige Katalysator wurde anschließend mit 365 ml einer wäßrigen, 40 g Vanadium als Vanadyloxalat enthaltenden Lösung getränkt. Die Trockung des Trägers im Warmluftstrom erfolgte wie oben angegeben. Anschließend wurde der Katalysator in einem Rohrofen bei 300°C 4 Stunden thermisch behandelt, wobei das Oxalat zersetzt wurde.

Abschließend wurde der Katalysator mit 365 ml einer wäßrigen, 14 g Blei als Bleiacetat und 14 g Rhenium als Re₂O₇ enthaltenden Lösung getränkt und wiederum im aufsteigenden Luftstrom getrocknet.

Der fertige Katalysator enthielt 40 g Pd, 40 g Vanadium, 14 g Blei und 14 g Rhenium pro Liter Träger.Der Katalysator wurde mit einem Liter unbehandeltem Trägermaterial gleichmäßig vermischt.

### Beispiele zur Nitrobenzolhydrierung

### Beispiel 3 (Vergleichsbeispiel)

In ein mit Öl thermostatisiertes Reaktorrohr mit einem Innendurchmesser von ca. 26 mm wurde eine 285 cm hohe Schüttung eines Katalysators hergestellt gemäß Beispiel 1 gefüllt. Der Katalysator wurde zuerst mit Stickstoff, dann mit Wasserstoff gespült und anschließend in einem Wasserstoffstrom von ca. 1 528 NL/h in 5 Stunden auf 240°C aufgeheizt. Anschließend wurde begonnen, Nitrobenzol im Wasserstoffstrom zu verdampfen. Die Mtrobenzol-Wasserstoff-Mischung gelangte mit ca. 230°C auf die Oberfläche der Katalysatorschüttung. Die spezifische Belastung des Katalysators wurde innerhalb von 80 Stunden von 0,2 auf 1,05 kg/l×h erhöht, was einer Flächenbelastung von 2994 kg/m²×h entspricht, so daß im Mittel eine Belastung von 1,03 kg/l×h erzielt wurde.

Im gesamten Versuch wurde darauf geachtet, daß der Katalysator an keiner Stelle wärmer als 440°C wird.

Die Öltemperatur wurde in 20°C-Schritten nach ca. 700, 800 und 900 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s.

Der Katalysator erreichte eine Standzeit von ca. 1 050 Stunden, nach denen der Nitrobenzol-Gehalt des Kondensates von 0 auf ca. 300 ppm anstieg und der Katalysator daraufhin durch Abbrennen regeneriert werden mußte.

Die durchschnittliche Selektivität betrug 99,0 %.
Im zweiten Zyklus nach der Regenerierung verhielt sich der Katalysator mit einer Standzeit von 990 Stunden und 99,2 % Selektivität unverändert.

### Beispiel 4

In ein mit Öl thermostatisiertes Reaktorrohr mit einem Innendurchmesser von ca. 26 mm wurde eine 285 cm hohe Schüttung eines Katalysators hergestellt und verdünnt gemäß Beispie 2 gefüllt. Der Katalysator wurde zuerst mit Stickstoff, dann mit Wasserstoff gespült und anschließend in einem Wasserstoffstrom von ca. 1528 NL/h in 5 Stunden auf 240°C aufgeheizt. Anschließend wurde begonnen, Nitrobenzol im Wasserstoffstrom zu verdampfen. Die Nitrobenzol-Wasserstoff-Mischung gelangte mit ca. 230°C auf die Oberfläche der Katalysatorschüttung. Die spezifische Belastung des Katalysators wurde innerhalb von 50 Stunden von 0,2 auf 1,07 kg/l×h erhöht, was einer Flächenbelastung von 3 051 kg/m²×h entspricht, so daß im Mittel eine Belastung von 1,03 kg/l×h erzielt wurde. Im gesamten Versuch wurde darauf geachtet, daß der Katalysator an keiner Stelle wärmer als 440°C wird.

Die Öltemperatur wurde in 10°C-Schritten nach ca. 266, 270, 293, 314, 317, und 362 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s.

Der Katalysator erreichte eine Standzeit von ca. 394 Stunden, nach denen der Nitrobenzol-Gehalt des Kondensates von 0 auf ca. 300 ppm anstieg und der Katalysator daraufhin durch Abbrennen regeneriert werden mußte.

Die durchschnittliche Selektivität betrug 99,78 %.
Im zweiten Zyklus nach der Regenerierung zeigte der Katalysator mit einer Standzeit von 876 Stunden und 99,7 % Selektivität eine deutliche Standzeitsteigerung.
Die Standzeitzunahme setzte sich im dritten und vierten Zyklus mit 898 und 997 Stunsen fort.

Der vierte Zyklus wurde in den ersten 220 Stunden mit N₂/H₂-Mischungen gefahren um die guten Anfahrselektivitäten noch weiter zu verbessern (siehe Tabelle 1).

**Tabelle 1**

| (Anfahrselektivitäten bei der Nitrobenzolhydrierung): | | | | | | |
|---|---|---|---|---|---|---|
| Gas | H₂/N₂ | | H₂ | | H₂ | |
| Versuch | Beispiel 4 | 4. Lauf | Beispiel 4 | 1. Lauf | Beispiel 3 | 1. Lauf |
| Katalysat | Beispiel 2 | | Beispiel 2 | | Beispiel 1 | |
| durch. Sel. | 99,7% | | 99,7% | | 99,0% | |
| Standzeit | 997 h | | 394 h | | 1052 h | |

| Laufzeit h | Belastung kg/L×h | Selekt. % | Belastung kg/L×h | Selekt. % | Belastung kg/L×h | Selekt. % |
|---|---|---|---|---|---|---|
| 3,5 | 0,31 | 98,0 | 0,24 | 90,4 | 0,27 | 43,5 |
| 24 | 0,50 | 98,5 | 0,53 | 97,4 | 0,50 | 67,3 |
| 48 | 0,60 | 98,4 | 0,71 | 99,2 | 0,80 | 83,0 |
| 72 | 0,65 | 98,9 | 0,98 | 99,6 | 1,10 | 93,9 |
| 94 | 0,74 | 99,2 | 1,00 | 99,8 | 1,10 | 97,5 |

### Beispiele zur o-Nitrotoluolhydrierung

### Beispiel 5 (Vergleichsbeispiel)

In ein mit Öl thermostatisiertes Reaktorrohr mit einem Innendurchmesser von ca. 33 mm wurde eine 285 cm hohe Schüttung eines Katalysators hergestellt gemäß Beispiel 1 gefüllt. Der Katalysator wurde zuerst mit Stickstoff, dann mit Wasserstoff gespült und anschließend in einem Wasserstoffstrom von ca. 1 560 NL/h in 5 Stunden auf 270°C aufgeheizt. Anschließend wurde begonnen, o-Nitrotoluol im Wasserstoffstrom zu verdampfen. Die o-Nitrotoluol-Wasserstoff-Mischung gelangte mit ca. 260°C auf die Oberfläche der Katalysatorschüttung. Die spezifische Belastung des Katalysators wurde innerhalb von 47 Stunden von 0,2 auf 0,62 kg/l×h erhöht, was einer Flächenbelastung von 1097 kg/m²×h entspricht, so daß im Mittel eine Belastung von 0,58 kg/l×h erzielt wurde. Im gesamten Versuch wurde darauf geachtet, daß der Katalysator an keiner Stelle wärmer als 440°C wird.

Die Änderung der Öltemperatur entlang des Reaktorrohres betrug ca. ±1°C. Die Strömungsgeschwindigkeit des Öles entlang der Rohroberfläche betrug ungefähr 1,5 m/s,

Der Katalysator erreichte eine Standzeit von ca. 216 Stunden, nach denen der o-Nitrotoluol-Gehalt des Kondensates von 0 auf ca. 300 ppm anstieg und der Katalysator daraufhin durch Abbrennen regeneriert werden mußte. Damit hat der Katalysator unter den angegebenen Randbedingungen eine >Produktivität von ca. 125 kg/l erreicht.
Die durchschnittliche Selektivität betrug 99,85 %.

Im zweiten Zyklus nach der Regenerierung verhielt sich der Katalysator mit einer Standzeit von 198 Stunden und 99,85 % Selektivität annähernd unverändert.

### Beispiel 6

In ein mit Öl thermostatisiertes Reaktorrohr mit einem Innendurchmesser von ca. 33 mm wurde eine 285 cm hohe Schüttung eines Katalysators hergestellt gemäß Beispiel 2 gefüllt. Der Katalysator wurde zuerst mit Stickstoff, dann mit Wasserstoff gespült und anschließend in einem Wasserstoffstrom von ca. 1560 NL/h in 5 Stunden auf 270°C aufgeheizt. Anschließend wurde begonnen, o-Nitrotoluol im Wasserstoffstrom zu verdampfen. Die o-Nitrotoluol-Wasserstoff-Mischung gelangte mit ca. 260°C auf die Oberfläche der Katalysatorschüttung. Die spezifische Belastung des Katalysators wurde innerhalb von 50 Stunden von 0,13 auf 0,689 kg/l×h erhöht, was einer Flächenbelastung von 1 219 kg/m²×h entspricht, so daß im Mittel eine Belastung von 0,644 kg/l×h erzielt wurde. Im gesamten Versuch wurde darauf geachtet, daß der Katalysator an keiner Stelle wärmer als 440°C wird.

Die Änderung der Öltemperatur entlang des Reaktorrohres betrug ca. ±1°C. Die Strömungsgeschwindigkeit des Öles entlang der Rohroberfläche betrug ungefähr 1,5 m/s. Der Katalysator erreichte eine Standzeit von ca. 239 Stunden, nach denen der o-Nitrotoluol-Gehalt des Kondensates von 0 auf ca. 300 ppm anstieg und der Katalysator daraufhin durch Abbrennen regeneriert werden mußte. Damit hat der Katalysator unter den angegebenen Randbedingungen eine >Produktivität von ca. 154 kg/l erreicht.
Die durchschnittliche Selektivität betrug 99,82 %.

Im zweiten Zyklus nach der Regenerierung zeigte der Katalysator mit einer Standzeit von 439 Stunden und 99,87 % Selektivität eine deutliche Standzeit steigerung.
Im zwölften Zyklus nach der elften Regenerierung erzielte der Katalysator eine Standzeit von 525 Stunden und eine durchschnittliche Selektivität von 99,9 %.

Der erfindungsgemäße Katalysator besitzt demnach im Untersuchungszeitraum über viele Regenerierungen eine gute Standzeit bei hoher Belastung.

## Patentansprüche

1. Verfahren zur Herstellung von aromatischen Aminen durch katalytische Hydrierung von entsprechenden aromatischen Nitroverbindungen in der Gasphase, **dadurch gekennzeichnet, daß** man die Hydrierung in Gegenwart von Katalysatoren, die neben Palladium, Vanadium und Blei noch zusätzlich Rhenium auf einen keramischen Träger mit einer BET-Oberfläche von weniger als 40 m³/g enthalten, bei Temperaturen von 180 bis 500°C im Katalysatorbett und einem molaren Verhältnis von Wasserstoff zur Nitrogruppe oder Nitrogruppen von 3:1 bis 30:1 durchführt.

2. Hydrierkatalysatoren, die neben Palladium, Vanadin und Blei noch zusätzlich Rhenium auf einem keramischen Träger mit einer BET-Oberfläche von weniger als 40 m³/g enthalten und wobei der Gehalt an Palladium 1 bis 50 g, der Gehalt an Vanadium 1 bis 50 g, der Gehalt an Blei 1 bis 20 g und der Gehalt an Rhenium insgesamt 1 bis 20 g pro Liter keramischen Trägermaterials beträgt.

## Claims

1. A process for the production of aromatic amines by catalytic hydrogenation of corresponding aromatic nitro compounds in the vapour phase, **characterised in that** the hydrogenation is carried out in the presence of catalysts which, besides palladium, vanadium and lead, contain in addition rhenium on a ceramic support having a BET surface area of less than 40 m³/g, at temperatures of from 180°C to 500°C in the catalyst bed and with a molar ratio of hydrogen to the nitro group or nitro groups of from 3:1 to 30:1.

2. Hydrogenation catalysts which, besides palladium, vanadium and lead, contain in addition rhenium on a ceramic support having a BET surface area of less than 40 m³/g and wherein the palladium content is from 1 to 50 g, the vanadium content is from 1 to 50 g, the lead content is from 1 to 20 g and the rhenium content is from 1 to 20 g per litre of ceramic support material.

## Revendications

1. Procédé pour la préparation d'amines aromatiques par hydrogénation catalytique des dérivés aromatiques nitrés correspondants en phase gazeuse, **caractérisé en ce que** l'on procède à l'hydrogénation en présence de catalyseurs contenant, avec du palladium, du vanadium et du plomb, du rhénium sur une matière de support céramique à surface BET inférieure à 40 m²/g, à des températures de 180 à 500°C dans le lit de catalyseur et avec un rapport molaire hydrogène/groupe nitro ou groupes nitro de 3:1 à 30:1.

2. Catalyseurs d'hydrogénation contenant, en plus du palladium, du vanadium et du plomb, du rhénium sur une matière de support céramique à surface BET inférieure à 40 m²/g, la teneur en palladium étant de 1 à 50 g, la teneur en vanadium de 1 à 50 g, la teneur en plomb de 1 à 20 g et la teneur en rhénium au total de 1 à 20 g par litre de matière de support céramique.
